# EUROPEAN PATENT APPLICATION

(11) **EP 3 579 241 A1**
(43) Date of publication of application: **11.12.2019**
(21) Application number: 18176732.8
(22) Date of filing: 08.06.2018
(51) Int. Cl.: G16H 10/60

(54) **METHOD OF MANAGING MEDICAL RECORDS**

(71) Applicant: Siemens Healthcare GmbH, 91052 Erlangen (DE)
(72) Inventor: ATHMANATHAN, Balakrishnan, 560021 Bangalore (IN); RANGAPURA SHETTAPPA, Chandrashekara, 91058 Erlangen (DE)

(57) **Abstract**

The invention describes a method of managing medical records (R), which method comprises the steps of obtaining medical data (D) from a source (11_1, 11_2, ..., 11_n); generating a medical record (R) comprising the medical data (D); augmenting the medical record (R) with metadata (Mᵣᵤₗₑ, M_{log}), wherein the metadata (Mᵣᵤₗₑ) comprises access permissions to that medical data (D) and/or a log file for that medical record (R); storing the medical record (R) in a cloud storage arrangement (10); and responding to a request for a medical record (R) on the basis of the metadata (Mᵣᵤₗₑ, M_{log}) of that medical record (R). The invention further describes medical record management system (1).

## Description

The invention describes a method of managing medical records, and a medical record management system.

Medical data collected by institutions such as hospitals is usually stored for at least several years. Because of the quantities of data that are generated in the course of routine examinations and by hospital records departments, storing this data can become a significant cost factor. Furthermore, in recent years there has been an explosive growth in medical digital medical information, for example because medical data can be significantly richer in content and may also reference related documents, resulting in a vast amount of medical information that must be managed. Local long-term storage of such large quantities of data is becoming a problem for the owner, e.g. a hospital, since the cost of providing and maintaining a local data centre can be significant. Small-scale storage of medical data is problematic for other reasons also. For example, medical data stored in a local data centre is effectively unavailable for research purposes, since access to the locally-stored data is limited to the owner. As a result, potentially valuable data is effectively not available to others for research and analytics purposes. Furthermore, older locally-stored data can effectively be "lost", for example when its format is no longer compatible with a newer format, or when older formats are no longer compatible with newer platforms, etc. One way of addressing these problems is to outsource data storage to a provider, for example to a cloud storage provider.

While this can make it easier for a hospital to manage long-term storage of large quantities of data, there are other problems associated with outsourcing data storage. It is the responsibility of the hospital to ensure compliance with any regulations and guidelines regarding access to medical data originating from patients of that hospital. However, when data storage is outsourced, there may not be any regulations in place to ensure safety of the data. If a hospital has a policy of sharing cloud-stored data with research institutions for the purpose of improving treatment methods, the hospital and those research institutions carry the responsibility of ensuring that the data is not released - intentionally or otherwise - to an unauthorized party. However, it has been observed that significant amounts of patient data are being shared across regions/countries without patient consent. This can be in direct violation of government regulations regarding data privacy, and legal action could be taken against a hospital/research institution for releasing medical data without proper authorization.

Equally, an organization requesting medical datasets for research and analytics purposes can run into legal problems. Such an organization generally has no way of knowing that a provider (the "source") of medical data has not taken measures to comply with data privacy regulations. An organization that uses patient data from an insecure source for research and analytics purposes can be severely penalized. Equally, a person may take legal action against an organization that - knowingly or unknowingly - used that person's medical data without permission.

It is therefore an object of the invention to provide an improved way of managing medical data to avoid the problems described above.

This object is achieved by the method of claim 1 of managing medical records; by the medical record management system of claim 11; and by the computer program product of claim 15.

According to the invention, the method of managing medical records comprises the steps of obtaining medical data from a source such as a hospital or clinic; generating a medical record comprising the medical data; augmenting the medical record with metadata, wherein the metadata defines access permissions to that medical data and/or wherein the metadata comprises a log file for that medical record; storing the medical record in a cloud storage arrangement; and responding to a request for a medical record on the basis of the metadata of that medical record.

The expression "medical data" as used in the context of the invention may be understood to comprise any kind of data such as patient information, images, laboratory reports, etc. For example, medical data may comprise one or more images generated by a medical imaging device such as a scanner, along with performance logs or machine data of the scanner. The expression "medical record" in the context of the invention should not be confused with the term "medical record" as used in a records department to refer to a patient's file. Instead, the expression "medical record" as used in the context of the invention may be understood to mean a collection of patient-related medical data as well as any additional information added to the medical data so that it can be stored in the cloud storage arrangement. A "medical record" as used in the context of the invention might therefore comprise a set of images following an imaging procedure as well as any relevant patient information, doctor's reports, etc., and augmented by the metadata described above.

In the following, it may be assumed that medical data may comprise unstructured data and/or structured data, and any number of files in various formats, for example cRSP (common Remote Service Platform) files generated for machine log data, HL7 (Health Level-7) files, portable data format (PDF) files, extensible markup language (XML) files, JavaScript object notation (JSON) etc. As indicated in the introduction, data can be uploaded from a source (e.g. a hospital) to a cloud storage platform, and the cloud storage platform may allow various approved institutions or organizations to access the data. Preferably, the metadata generated for medical data is embedded in a file of the medical record. Metadata can be added to the medical data in the form of tags, and may be embedded in a suitable file of the medical record. For example, metadata tags can be added to a cRSP file, a JSON file, etc. An advantage of the inventive system is that the metadata becomes part of each medical record. In other words, once a medical record has been created by augmenting medical data with metadata, the medical data is effectively inseparable from the metadata. Thereafter, access to a medical record is approved or denied on the basis of the metadata of that medical record.

The inventive medical record management system provides a way of storing essentially unlimited numbers of medical records in a cloud storage arrangement. The vast quantity of data has potentially great value for research and analytics purposes, and can serve as the basis from which to learn how to improve treatment of a condition, for example. A request to access certain datasets may be received from a requesting party such as a research institute, an analytics institute, etc. The request may be to view, copy or download certain types of medical data.

An advantage of an embodiment of the inventive method is that access permissions for medical data can be stored as metadata of a medical record, so that the owner or data source (e.g. a hospital) can be assured that it has taken all necessary measures to comply with any applicable regulations, while a requesting party (e.g. an R&D institution, a teaching university, etc.) can be certain that any requested and received medical data has originated from a secure source. The nature of the access permissions will be explained in more detail below. In the context of the invention, it shall be understood that a medical record is only ever released or shared following an approved request, i.e. medical data will only be released if the access permissions recorded in the metadata of the medical record are compatible with the requesting party. As explained above, in the prior art systems a requesting party may unwittingly obtain access to records that should in fact not have been released, and may face legal consequences as a result. An advantage of an embodiment of the inventive method is that any request for medical data will return data only from medical records that have the appropriate access permissions.

A further advantage of an embodiment of the inventive method is that it can assist in auditing procedures by using the log files of the medical records to record any access request to a medical record. The log files may be used to demonstrate that requests for a medical record were turned down if the access rights for that medical record were incompatible with the requesting party, for example.

Since the medical records are stored in a cloud storage arrangement and may be shared within or released from that cloud storage arrangement, the inventive method may be referred to in the following as a cloud-based method of managing medical records.

According to the invention, the medical record management system comprises a means of receiving medical data to be stored in a medical record; a metadata generator realised to augment the medical data with metadata defining access permissions to that medical data and/or comprising a log file for that medical data; a cloud storage arrangement for storing the medical data with its metadata as a medical record; and a request manager realised to receive a request for medical data and to respond to the request on the basis of metadata of a corresponding medical record.

An advantage of the inventive cloud-based medical record management system is that it provides a straightforward way of ensuring that medical records are treated in a secure manner. Since a medical record comprises the medical data as well as the metadata relating to the medical data, a medical record stored in the inventive medical record management system may be regarded as "self-contained" in the sense that there is no need to consult any other information source to find out the access permissions for that medical record and/or to see who has requested that medical record.

According to the invention, the computer program product comprises a computer program that is directly loadable into a memory of a control unit of the inventive medical record management system and which comprises program elements for performing steps of the method according to any of claims 1 to 10 when the computer program is executed by the control unit of the medical record management system. A memory can be realised as a non-permanent main memory (e.g. a random-access memory) or in the form of a permanent mass storage device (e.g. a hard disk, a USB stick, an SD card, a solid-state storage device, etc.).

The units or modules of the inventive medical record management system mentioned above can be completely or partially realised as software modules running on a computation unit such as a processor, an FPGA, an ASIC, etc. A realisation largely in the form of software modules can have the advantage that applications already installed on an existing system can be updated with relatively little effort.

Preferably, a computer readable medium such as a memory stick, a hard-disk or other transportable or permanently-installed carrier is provided to transport and/or to store the executable parts of the inventive computer program product so that these can be read from a processor unit of a medical record management system. A processor unit can comprise one or more microprocessors or their equivalents. In particular, program elements are stored on a computer-readable medium, wherein the program elements can be read and executed by a medical record management system, in order to perform steps of the method for method of managing medical records according to one of the claims 1 to 10, when the program elements are executed by the medical record management system and/or a processor unit of a medical record management system.

The inventive medical record management system can be executed on one or more computation units or computers. The inventive medical record management system can run on a real group of computers (a "cluster") and/or on a virtual group of computers (a "cloud"). A computer can be any of a personal computer, a workstation, a virtual machine running on host hardware, a microcontroller, an integrated circuit, etc.

Particularly advantageous embodiments and features of the invention are given by the dependent claims, as revealed in the following description. Features of different claim categories may be combined as appropriate to give further embodiments not described herein.

In the following, it may be assumed that the cloud storage arrangement is realised for global use, i.e. medical data can be uploaded from any country worldwide and may potentially be accessed by a requesting party from any country. As mentioned above, medical data can originate from any source such as a hospital or clinic. In the following, without restricting the invention in any way, it may be assumed that medical data originates from a hospital.

Without restricting the invention in any way, it may be assumed that the medical record management system comprises an interface for uploading data to a cloud storage arrangement in a data transfer step. The interface may be realised as a web interface, for example. Here and in the following, an "interface" may be understood to comprise a hardware interface such as a PCI-bus interface, a USB interface, an IEEE 1394 interface, etc. An interface may comprise software elements or may be realised entirely in the form of software.

A data transfer can comprise any amount and any type of data. For example, a data transfer may comprise all information relevant to a medical imaging procedure. Such information may comprise patient data (identification, medical insurance information etc.) as well as any medical data relating to the imaging procedure (images, imaging protocol used for the imaging procedure, radiation dose absorbed by the patient, laboratory reports, doctor's reports, etc.). Machine logs from the imaging apparatus may also be included as part of the information uploaded to the cloud storage arrangement. For example, a cRSP file can be a machine log file generated by a medical apparatus such as an imaging device during a task such as an imaging procedure.

Some of this data may be considered sensitive, for example any information that might allow identification of the actual patient. Such sensitive information might comprise any details about the patient's health status, healthcare record, insurance company, etc. that can be linked to that specific individual. This kind of sensitive information may be referred to as protected health information (PHI). Another type of sensitive data might be classified information, for example any medical data relating to military personnel or originating from a military hospital. In a preferred embodiment of the invention, therefore, the inventive medical record management system comprises a means for performing data separation, so that any sensitive data is initially identified and separated from the remainder of the medical data, which may be referred to as the non-sensitive data. For example, medical images that cannot be related in any way to a particular individual may be regarded as non-sensitive and may be shared (under certain circumstances) with a requesting party.

To ensure that medical information cannot be related in any way to a particular individual, the inventive medical record management system preferably also comprises a means for performing anonymization of the sensitive information. Preferably, the step of anonymizing sensitive data is performed so that any relevant entry is altered in such a way that it is not possible for a requesting party to relate the non-sensitive data with the sensitive data. The data separation module can separate and anonymise any sensitive and/or classified data from non-sensitive data of a medical record. Preferably, the inventive medical record management system also comprises an encryption module that is realised to encrypt any sensitive and/or classified data. In a preferred embodiment of the invention, all identification details (patient name, age, gender, date of birth, etc.) are preferably saved as an encrypted JSON file.

In such a preferred embodiment of the invention, sensitive data is separated from other data and encrypted, so that all elements of a medical record are securely stored in the cloud. An advantage of this type of data separation is that the non-encrypted data can be viewed by any approved requesting party from anywhere in the world, while at the same time ensuring patient privacy by the segregation and encryption of sensitive data. This approach makes it easy for hospitals to share information such as images and clinical findings for research and analytics without violating data privacy.

As indicated above, a medical record can have any combination of structured data, unstructured data, image files, laboratory reports, machine performance logs, etc. For example, it may be more appropriate to store a large quantity of unstructured data as a binary large object ("blob"). Structured data, on the other hand, may be more efficiently stored in a structural database. In a preferred embodiment of the invention, therefore, the medical record management system comprises a means for choosing a suitable data storage policy for a medical record. For example, elements of the medical record that may be expected to be accessed relatively often (active and/or dynamic content) may be stored in a manner suited to relatively frequent retrieval, while elements of the medical record that will most likely never or rarely retrieved (inactive and/or fixed content) may be placed in "cold storage".

As indicated above, a hospital may have a policy of sharing data with one or more requesting parties, for example with certain research institutes. At the same time, a patient at that hospital may specify that his/her data is not to be shared with any outside party, thereby ruling out the possibility of that hospital sharing that patient's data with any requesting party. In a preferred embodiment, the inventive medical records management system comprises an input interface realised to specify an access scope for medical data at the patient access level, i.e. the access level at which a patient can record her intentions relating to access to her medical data. For example, after admission to a hospital and prior to a medical procedure, a patient is requested to fill out a patient consignment form specifying any preferences regarding data privacy. A patient consignment form can appear as a number of fields or check-boxes which the patient can fill out and sign, thus giving or withholding approval to share data. The set of preferences specified by a patient may be referred to as the "patient consignment". The input interface preferably also comprises a rule set generator that is realised to generate a rule set on the basis of the patient consignment. In a preferred embodiment of the invention, a patient consignment specifies at least an approved use of the medical data, i.e. which elements of that patient's data may be shared and the type of requesting party that may be given access to those elements. For example, a patient may agree to research institutes being given access to his or her medical data, but may not wish to give such permission to any corporate analytics institute.

The input interface is preferably realised as a web-based application for ease of use. The same web application can be used by a hospital to initiate an upload of medical data to the cloud. Preferably, the web-based application is realised to access the data from the cloud through the interface and might also be used by any institution that needs medical data for research purposes, analytics purposes, etc.

In a preferred embodiment of the invention, access permissions for a medical record are defined by several rule sets, wherein a rule set defines a scope of access to medical data of that medical record at a specific access level. In the inventive method, such rule sets are stored as metadata and therefore become part of the medical record. As explained above, one rule set may define the scope of access desired by the patient. A separate rule set or "hospital consignment" may define the scope of access permitted by the hospital. In a preferred embodiment of the invention, a hospital consignment specifies one or more of: an approved use of the medical data, the country of origin of the medical data, the storage duration of the medical data, the owner of the medical data, etc. A hospital consignment may also include rules relating to hospital policy, for example the sharing of medical data with family members of a patient; the sharing of medical data with insurance companies for payment purposes; a list of regulation-compliant authorized dealers for the procurement of healthcare items, etc. A rule set generator preferably generates a rule set on the basis of the hospital consignment. Here also, an input interface can be provided in the form of a web application for ease of use by hospital personnel.

A hospital in a specific country may need to comply with certain legal regulations regarding privacy and data security that apply to that country. Therefore, in a further preferred embodiment of the invention, a rule set or "country consignment" may define the scope of access allowed by the country in which the hospital is based. Such rules may relate to the disclosure of medical data when required by national/regional law; disclosure of medical data in compliance with a court order, etc. A member country (e.g. Germany) of a region (e.g. the European Union) may in turn need to comply with certain legal regulations regarding privacy and data security that apply to that region. Therefore, in a further preferred embodiment of the invention, a rule set or "region consignment" may define the scope of access allowed by the region of which the country (in which the hospital is based) is a member.

Other regulations may apply to the manufacture of medical devices to ensure patient safety. By including such information in the country/region consignment, a requesting party can specify data that originates only from devices that comply with such regulations.

Preferably, the metadata generator comprises at least one regulation module realised to augment a medical record with regulation metadata. Any relevant regulations, stipulations, requirements etc. at a specific access level may be laid out in one or more contracts or similar documents. In a particularly preferred embodiment of the invention, therefore, a metadata generator preferably comprises a parser to identify any relevant regulations, stipulations, or requirements that are laid out in such a document.

In a preferred embodiment of the invention, the medical records management system comprises a regulation module realised to augment a medical record with regulation metadata pertaining to the patient consignment and/or hospital consignment (i.e. regulation metadata relating to the patient and/or hospital). At this level (i.e. the hospital or "owner" level), some relevant regulations, stipulations, requirements etc. may be defined in the clauses of a service contract. Preferably, a regulation module comprise a parser that is configured to recognise and extract relevant regulatory information from documents such as service contracts, legal contracts, etc., so that the regulation module may be referred to as a "legal and regulatory manager" at hospital level.

In a further preferred embodiment of the invention, the medical records management system comprises a second regulation module realised to augment a medical record with regulation metadata pertaining to the country consignment and/or region consignment, e.g. regulation metadata pertaining to national and/or regional requirements. The advantage of this is that it is not necessary for hospital personnel to have to deal with details relating to the national/regional regulations, and the responsibility for this task can be moved to the level of the cloud-based system.

In a particularly preferred embodiment of the invention, medical data of a requested medical record is released to a requesting party only if the requesting party meets the requirements specified in the metadata of that medical record. In other words, a medical record originating in a hospital in a specific country will only be released to a requesting party if such release is in compliance with the access permissions as defined in the metadata of the medical record. For example, medical records originating in a hospital in a country X will not be released to any requesting party from country Y if the relevant region (to which the country X belongs) does not permit sharing of medical data with country Y.

As explained above, a rule set defines access rules that apply to the medical data and may be laid down by the patient and/or the hospital and/or the country and/or the region, so that access to the medical data of a medical record can be defined at various specific levels: at one level, medical data may (or may not) be released to a requesting party from a particular country; at another level, a patient may have specified that certain components of his medical data may not be released to any requesting party, regardless of the rule sets at different levels etc. Preferably, a rule set comprises at least a patient consignment, a hospital consignment, a country consignment, and a region consignment. While a region consignment takes precedence over a country consignment (and hospital consignment), the patient consignment may take precedence over any other consignment, specifically as regards sharing of medical images, doctor's reports, laboratory reports, etc. Each self-contained medical record effectively "decides" by whom and to what extent it may be accessed.

In a preferred embodiment of the invention, the request manager is configured to analyse the metadata of (relevant) stored medical records in response to a request for a specific type of medical data, to identify any approvable medical records, i.e. medical records that match the request and that may be accessed, and to release medical data of the approved medical records to the requesting party.

Following a request for a certain kind of data, for example basal ganglia data from all hospitals in the USA, data from matching medical records may be identified and collected in a repository. To this end, the request manager of the medical records management system preferably comprises a rule analyser (also referred to as "regulation analyser" or "policy analyser") that identifies any medical record that matches the request and which may be released to the requesting party. For example, if that requesting party is an analytics institute in the USA, the regulation analyser does not need to be concerned with the region consignment and country consignment, and can focus on the hospital consignment and patient consignment to identify any relevant medical record which also has the relevant permission (for example both hospital consignment and patient consignment say "willing to share with analytics institutes").

However, a requesting party can be anywhere in the world. Therefore, if the requesting party is an analytics institute in a different country than the country or region from which it is requesting information, the regulation analyser first checks the region consignment and country consignment to see if the access request is at all legitimate. Only when the access request is considered legitimate (i.e. can be approved in principle) will the regulation analyser proceed to the level of hospital consignment, and then, after identifying hospitals that are willing to share with an analytics institute, proceed to the level of patient consignment. The datasets identified by the regulation analyser are filtered by a requirement matcher so that only data that is of interest to the requesting party will actually be stored in a repository.

As explained above, in an embodiment of the inventive method, the metadata comprises a log file for the medical data so that all access requests can be recorded, whether or not those access requests were approved. Preferably, the inventive method comprises a step of adding a log entry to the log file of a medical record in response to an action relating to that medical record. Such a log entry can comprise at least a description of the action (e.g. downloaded, updated, deleted, etc.) and/or an identifier of the party that initiated the action, etc. In this way, each request to access a medical record is recorded and stored as part of the log file. The log file will therefore contain a complete history for the medical record from the time of its creation.

Another important task that must be carried out reliably is the thorough deletion of a medical record if the occasion arises. For example, a medical record may approach the end of its retention period (e.g. ten years) and should be deleted completely. The relevant hospital can initiate deletion or destruction of the medical record by issuing an appropriate command to the inventive medical record management system, which can then retrieve the medical record and examine its log file to see if any requesting party was ever given access to copy the medical data. If so, the medical record management system will inform each requesting party to delete any local copies and to provide confirmation of their deletion. If the medical data of a medical record was never copied outside of the cloud storage system, any instance of the data can easily be deleted from the cloud storage system, including any repository into which the medical data may have been copied.

A medical record log file can also support an auditing process. For example, a hospital undergoing auditing may be expected to prove that it has ensured protection of its medical data. The hospital can retrieve the log files of all medical records and show that all unauthorized requesting parties were turned down, that medical data was only released to authorized requesting parties, that medical records were completely destructed upon expiry of their retention periods, etc.

A requesting party may typically request several hundreds or even thousand datasets relating to a specific topic, since a wide spread of data can be more useful for research or analytics purposes. In prior art cloud-based storage systems, it can be cost-intensive to search or trawl a database in order to identify relevant datasets. Furthermore, release of datasets may even be in violation of regulations or access permissions. Therefore, in a particularly preferred embodiment of the invention, prior to storing a medical record in the cloud storage arrangement, the method further comprises a step of analysing the content of the medical data and indexing the medical record on the basis of its content. The step of analysing the content of the medical data is preferably performed using a trained machine learning algorithm, for example using a deep neural network, a deep convolutional neural network, etc. Alternatively or in addition, medical data content analysis may be done manually or in a semi-automated manner. A medical record can be augmented with any number of index fields. For example, index fields may be assigned for one or more of the following: intended use of the medical data; duration of storage; access limitations; presence of sensitive data; owner of the data; storage time of the data; upload time of the data, etc. Another index field may be used to indicate that the medical record contains protected health information. Another index field may indicate the region of origin. Preferably, as mentioned above, the inventive system comprises a requirement matcher module that filters or matches medical data to a request. In a preferred embodiment of the invention, the requirement matcher uses the index values of a medical record to quickly decide whether or not the medical data is of any relevance to the requesting party.

By indexing each medical record according to its access permissions, a requesting party can be certain that access to the data is in full compliance with all applicable regulations and access permissions, and the owner of the data can be certain that the data will not be released to an unauthorized party.

Other index fields may be used to relate a medical record to a specific organ. In a preferred embodiment, a first index specifies the organ and a second index specifies an area of interest in that organ. An appropriate index combination can then be assigned to any medical record that includes medical data relating to a specific organ. For example, the index label "brain" can be added to all medical records that include medical data relating to the brain; the index label "heart" can be added to all medical records that include medical data relating to the heart, etc.

Specific areas of interest of an organ (i.e. parts of an organ that are often the subject of research) can be assigned an index number. An appropriate index number can then be added to any medical record that includes medical data relating to a specific area of interest. In the case of the brain, areas of interest may be the basal ganglia, the cerebellum, the occipital lobe, the brain stem etc. For example, if index number "1" relates to "basal ganglia", all medical records that include medical data relating to basal ganglia will be given index label "brain" and index number "1". Specific areas of interest of the heart may be left ventricle, right ventricle, aorta, etc. In the inventive method, all medical records that include medical data relating to the aorta can be given index label "heart" and index number "3", etc.

In a particularly preferred embodiment of the invention, the method comprises a step of generating a master index for the medical records stored in the cloud storage arrangement. As mentioned above, the cloud storage arrangement is preferably realised for global use, so that medical records can be uploaded from any country worldwide and may potentially be accessed by any number of requesting parties from any country. Such a master index is therefore particularly useful since it can greatly facilitate quick access to medical records that are stored in a distributed manner across multiple regions. The master index preferably comprises a database with an entry for medical record, specifying the organ(s), the area(s) of interest, the region of origin, the country of origin, etc.

As mentioned above, the cloud storage arrangement may be distributed over several different regions with different region regulations, and for this reason the inventive medical records management system preferably provides separate repositories for the different regions. A master index may be created and maintained for each region. For example, a master index for the "Europe" repository may comprise entries for each medical record originating from the European region, and each entry can have further index values indicating the content of the corresponding medical record, whether it can be shared outside the Europe region, etc.

In a preferred embodiment of the invention, processing of the medical data can also take place in the cloud. This can favourably reduce overheads for research institutions that would otherwise have to provide their own data storage capacities as well as their own computing capacities. Since the data never leaves the cloud storage arrangement but instead is processed from the repository, it is easier for the data owners to comply with regulations regarding the sharing or release of patient data.

It is not uncommon for a hospital to perform a weekly backup of all its digital medical data. A hospital may also perform a daily incremental backup so that day-to-day changes may be recorded. Storing such data in a cloud storage arrangement an organized manner as described above using the inventive method and system makes it relatively easy to ensure that back-ups are made at the desired rate.

As mentioned above, medical data generated in a hospital may originate in part from medical apparatus such as imaging systems, and the medical data may therefore also comprise machine data or machine logs (sometimes also referred to as performance logs). Such machine logs can record every action taken by the apparatus in the course of an imaging procedure, for example. In a preferred embodiment of the invention, the method comprises a step of analysing machine data of medical apparatus, for example to detect any potential violation. A violation may arise, for example, when a medical apparatus has been used in a manner that is not in keeping with the terms of use agreed between the medical apparatus supplier and the customer. To this end, the inventive medical records management system preferably also comprises a regulation input interface that provides any relevant legal/regulatory regulations in a machine-readable file format. To extract any relevant information from the machine data input, the inventive system preferably also comprises a parser that is realised to parse the input. Preferably, the inventive system also comprises a machine learning arrangement that is realised to recognise expected content and to identify any unexpected content. For example, the machine learning arrangement can apply any suitable pattern recognition technique to the analysis of the machine data. The machine learning arrangement preferably uses artificial intelligence, for example a neural network, to perform these tasks.

A service contract may define various aspects including the duration after which machine data must be deleted; the duration after which application files must be deleted; archiving regulations for machine/application files; access permissions to machine/application files etc. In a preferred embodiment of the invention, metadata relating to such legal/regulatory information is added at source to any such machine/application file. If any action (e.g. delete, download, share, etc.) is performed on such a file, the action will be checked against the rules, and any violation will be logged. If deemed relevant, a report about the suspected violation may be sent to the relevant administrator indicating an estimated hazard level.

Such an automated system that can identify abnormal usage can greatly reduce the workload of legal regulations and compliance officers, who would otherwise have to manually examine audit reports to identify improper access.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Fig 1 shows a simplified schematic of an embodiment of the inventive cloud-based medical record management system;
Fig 2 shows a simplified schematic of an embodiment of the inventive cloud-based medical record management system;
Fig 3 shows a cloud storage arrangement set up to store medical records originating from several regions;
Fig 4 shows an alternative representation of an embodiment of the inventive medical record management system;
Figs 5 - 7 are flowcharts that illustrate various aspects of the invention.

In the diagrams, like numbers refer to like objects throughout. Objects in the diagrams are not necessarily drawn to scale.

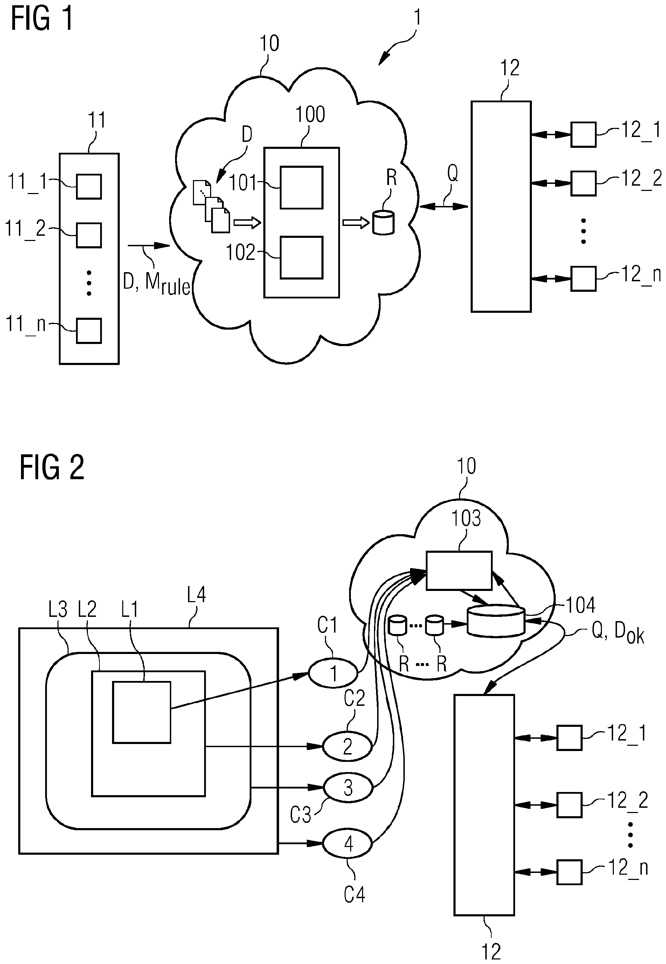

Fig 1 shows a very simplified schematic of an embodiment of the inventive cloud-based medical record management system 1, comprising a cloud storage arrangement 10, a hospital environment 11 with various sources 11_1, 11_2, ..., 11_n of medical data D, and a request interface 12 though which requesting parties 12_1, 12_2, ..., 12_n may be given access to medical data.

A source of medical data D can be a medical device 11_1, 11_2, ..., 11_n such as an MRI scanner, a CT scanner, etc. that generates application data during use, or simply a terminal by means of which a user (e.g. hospital personnel, doctor, etc.) can enter medical data D that is to be stored in the cloud storage arrangement 10. Medical data D can be uploaded to the cloud storage arrangement 10 in any suitable manner as will be known to the skilled person. The medical data D can be a dataset comprising any number of files, and these may have any file type. For example, medical data can comprise unstructured data and/or structured data. A dataset can include any number of files in various formats, for example cRSP, HL7, PDF, XML, JSON, etc. At the time of upload from a hospital to the cloud, the data may already be accompanied by metadata Mᵣᵤₗₑ for a patient consignment and/or a hospital consignment.

The medical record management system 1 further comprises an augmentation module 100 realised to augment the medical data D with further metadata. As will be explained in more detail below, additional metadata Mᵣᵤₗₑ can comprise a set of country/region access permissions to that medical data D and/or a log file for that medical data D. To this end, the augmentation module 100 comprises a rule metadata module 101 and/or a log metadata module 102. All metadata Mᵣᵤₗₑ is embedded with the medical data D to give a self-contained medical record R. The diagram only shows an exemplary dataset D and a single medical record R, but it shall be understood that the cloud storage arrangement 10 can store unlimited numbers of medical records R.

The diagram also shows only a single hospital environment 11, but it shall be understood that there is no limit to the number of customers or clients that upload data D for storage in the cloud 10. Since the cloud storage arrangement is realised for global use, medical data D can be uploaded from any country in the world. The medical data stored in the cloud 10 may potentially be accessed by any requesting party from any country, and access is managed through the request interface 12.

As will be explained below, the inventive medical record management system also comprises a request manager (not shown here) that receives and processes requests Q for medical data D and which responds to such requests Q on the basis of the metadata of corresponding medical records R. A requesting party 12_1, 12_2, ..., 12_n will only be given access to medical data D of medical records R that are allowed to be released to that requesting party.

This is illustrated in Fig 2. Here, the medical record management system 1 receives a request Q from a requesting party 12_1, 12_2, ..., 12_n. In this exemplary embodiment, it may be assumed that the request specifies a particular organ, an area of interest, preferred sources and a preferred region, for example {"heart"; "left ventricle"; "all hospitals"; "Europe"}. The task of identifying any matching datasets is easily achieved in the inventive method. To this end, a metadata analyser 103 or requirement matcher 103 is realised to examine the metadata of any number of medical records R stored in the cloud. Each of these medical records R contains metadata comprising rule sets or consignments C1, C2, C3, C4 for four access levels, shown on the left as a nested group L1, L2, L3, L4.

A first access level L1 corresponds to the patient and a patient consignment C1 defines the patient's wishes. At any time, a patient can be given the opportunity to specify the extent to which his or her data may be shared. The patient can specify his or her wishes by means of a web interface that appears as a form with checkboxes to tick or data fields to fill out, as appropriate. The scope of access or patient consignment C1 may extend from entirely private (the patient explicitly does not wish any data to be given to any requesting party, so that only the hospital may use his or her data) to entirely public (the patient agrees to allow access to any requesting party). For example, the patient consignment C1 may specify one or more of the following: countries/regions that may be given access; the type of requesting party that may be given access (e.g. "yes" to research & development institutions, "no" to business analytics institutes); whether the data may be downloaded or only viewed, etc. The patient consignment C1 may be included as part of the medical data uploaded to the cloud 10, and may already be included as metadata, or in a manner that can be converted to metadata by the metadata augmentation module 100.

A second access level L2 corresponds to the hospital and a hospital consignment C2 defines the hospital policy. Since hospital policy is generally well-defined from the outset, any relevant access rule may be obtained (automatically) from the relevant hospital regulations and added to patient data that is to be stored in the cloud. Of course, hospital personnel may also add or specify information for the hospital consignment C2. The hospital consignment C2 may specify one or more of the following: to which countries/regions data can be shared; policy regarding data sharing with patient's family members; policy regarding data sharing with patient's insurance company; policy regarding procurement supply chain, etc. Here also, the hospital consignment C2 may comprise part of the medical data to be uploaded to the cloud 10 and may already be included as metadata, or in a manner that can be converted to metadata by the metadata augmentation module 100.

A third access level L3 corresponds to the country in which the hospital is based and the country consignment C3 defines any national regulations that apply. The country consignment C3 may specify one or more of the following: to which countries/regions data can be shared; national regulations regarding patient data disclosure in compliance with court order; national regulations regarding mandatory reporting of births/deaths/contagious disease, etc. The metadata augmentation module 100 is realised to augment the medical data with the country consignment C3 in the form of metadata.

A fourth access level L4 corresponds to the region of which the country is a member state, and the region consignment C4 defines any applicable regional regulations. The region consignment C4 may be expected to be similar to the country consignment C3 and may differ from this only if there are regional regulations that are different from any member country regulations. For example, a European policy regarding patient data disclosure may differ from the policy of a member country. The metadata augmentation module 100 is realised to augment the medical data with the region consignment C4 in the form of metadata.

When a requesting party submits a request Q for a certain type of data from a certain region/country, the metadata analysis module 103 or requirement matcher can identify any medical record that matches the request (i.e. will be of relevance to the requesting party) AND may be viewed by or shared with the requesting party. Essentially, all levels L1, ..., L4 must be willing to share data with the requesting party in order for the medical data of a medical record to be released to that requesting party. Such records R (or their relevant medical data content) may be copied to a repository 104 to which the requesting party has access. If any one level (e.g. the hospital) denies access to a certain type of requesting party or to any requesting parties from a certain region, access to that medical record is denied to the requesting party, and medical data of the relevant record is not copied to the repository 104.

To facilitate quick identification of suitable medical records in response to a request, in a preferred embodiment of the invention the metadata augmentation module 100 is realised to add index tags to each medical record. For example, a medical record can be given a region index (e.g. "EU" when the medical record originates in Europe; "AF" when the medical record originates in Africa, etc.); a source index (e.g. an identifier that uniquely identifies the hospital that supplied the record); an organ label or organ index (e.g. "heart", "brain", "endocrine", etc.); and an area-of-interest index that identifies an area of interest of the organ (e.g. "1" for left ventricle if the organ index is "heart", etc.). A requesting party can then formulate a request using a string of appropriate index values, for example "EU all heart 1" to request data relating to the heart, specifically to the left ventricle, originating from all hospitals in the European region.

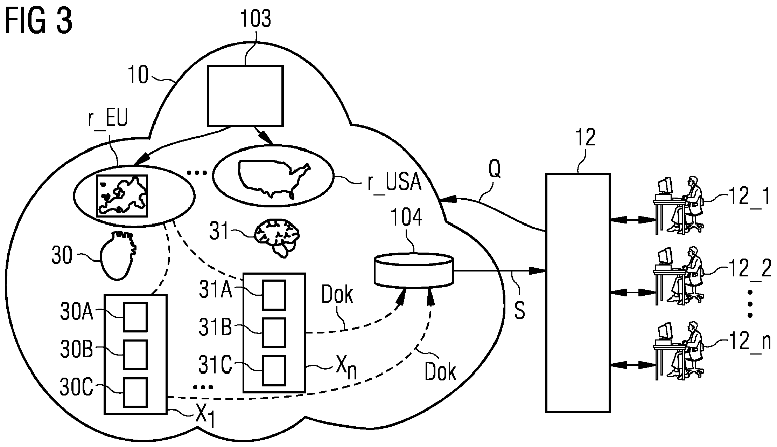

Fig 3 illustrates this principle in more detail. The diagram shows a cloud storage arrangement 10 set up to store medical records originating from multiple regions, of which two r_EU, r_USA are shown here. In each region, a master index X₁, ..., Xₙ has been compiled for each organ. The diagram shows two exemplary organs - heart 30 and brain 31, and two exemplary master indexes X₁, Xₙ. Here, the master index X₁ for region r_EU relates all medical records of the Europe region that contain data relating to the heart, identified according to area of interest, for example the left ventricle 30A, right ventricle 30B, aorta 30C, etc. Similarly, the master index Xₙ for region r_EU relates all medical records of the Europe region that contain data relating to the brain, identified according to area of interest, for example the basal ganglia 31A, front cortex 31B, visual cortex 31C, etc. It may be assumed that such master indexes are compiled for each region.

A first requesting party 12_1, ..., 12_n submits a request Q for a certain type of data from the European region r_EU, for example to view cardiac left ventricle images collected in any European hospital. A second requesting party 12_1, ..., 12_n submits a request Q for a certain type of data from any hospital in the United States of America region r_USA, for example to view brain basal ganglia data collected in any hospital in the USA.

In each case, the metadata analysis module 103 or requirement matcher can identify any medical record that matches the request (i.e. will be of relevance to the requesting party) AND may be viewed by or shared with the requesting party, simply by scanning the master index. Any matching and viewable data D_{OK} is copied to a repository 104 to which the requesting party has access. Data processing can be carried out on the approved data D_{OK} in the repository and the results S returned to the requesting party; alternatively, the requesting party may be given copies of the approved data D_{OK}. The repository is configured in such a way that a requesting party can only "see" data that it has requested, and cannot "see" data that has been requested by others. Medical data from records that do not match the search query or whose access rules deny permission to the requesting party will remain invisible to the requesting party.

In this way, a hospital that uploads data to the cloud can be sure that no unauthorized party will be given access if any of the hospital/country/region consignments forbids such access. A patient can be sure that his data will not be shared against his will. A requesting party can be sure that data is legitimately shared or released in response to a request, and need not be concerned about violating any data privacy regulations.

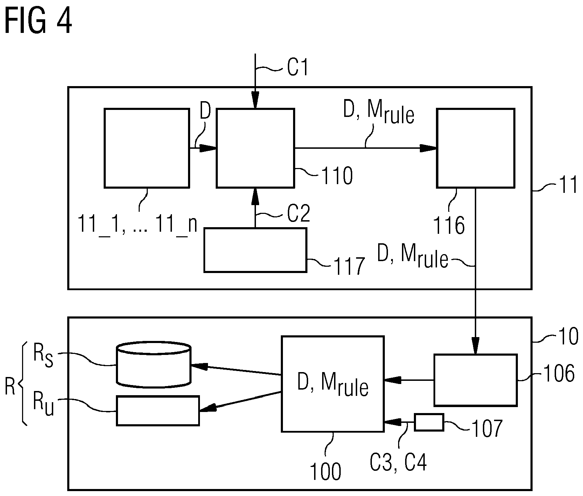

Fig 4 is an alternative representation of an embodiment of the inventive medical record management system 1. Here, data is generated by a medical device 11_1, 11_2, ..., 11_n in a hospital environment 11. The data can comprise one or more files of any type as explained above. Using an appropriate interface 110, for example a web interface 110, a first regulation module 117 adds metadata Mᵣᵤₗₑ covering the hospital consignment C2 to the data D generated by the medical device. The hospital consignment C2 can specify any regulations with which the hospital must comply and/or regulations defined in the hospital policy as regards data privacy and data sharing. The first regulation module 117 can be realised to parse any suitable machine-readable file that lays out relevant legal requirements, for example a service contract of the hospital. The input interface 110 may also be available to a patient, allowing at the patient to specify how his/her data may be accessed, so that the metadata Mᵣᵤₗₑ also includes patient consignment access rules C1.

The augmented data D, Mᵣᵤₗₑ is then given to a data upload means 116 for transfer to the cloud storage arrangement 10. In the cloud storage arrangement 10, a data receiving means 106 passes the uploaded information to a metadata augmentation module 100 that augments the data D with further metadata Mᵣᵤₗₑ according to any national/regional regulations as regards data privacy and data sharing. Here also, the metadata augmentation module 100 may comprise a further regulation module 107 that can provides the relevant legal requirements Mᵣᵤₗₑ, for example a list of up-to-date regional/national regulations Mᵣᵤₗₑ. The metadata Mᵣᵤₗₑ now includes access rules Mᵣᵤₗₑ for all four levels described in Fig 2 above, and the result is a medical record R that includes the medical data D and the embedded metadata Mᵣᵤₗₑ.

Depending on the nature of the medical data D and the file types that are included, a decision can then be made as to how the medical record R should be stored. For example, a medical record R may comprise both structured data R_{S} and unstructured data R_{U}, and different storage policies may be chosen for different elements of a medical record. Unstructured data R_{U} may be stored as a binary large object ("blob") while structured data R_{S} may be stored in a relational database.

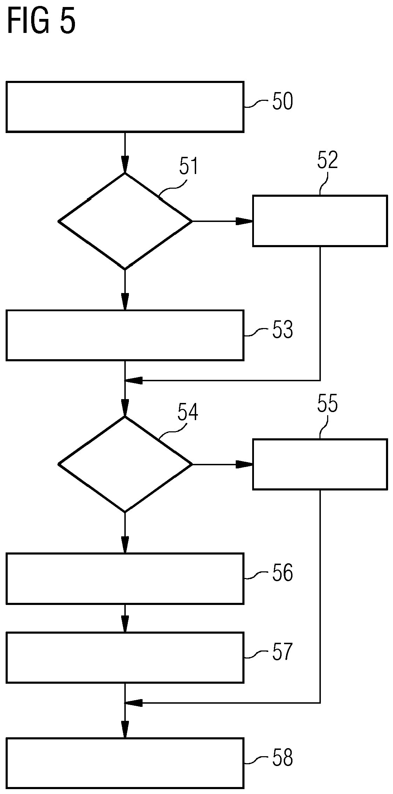

Figs 5 - 7 are flowcharts that illustrate various aspects of the invention:
Fig 5 is a flowchart that describes the steps taken to select a storage policy and to anonymise sensitive data. In a first step 50, a data receiving means (e.g. data receiving means 106 in Fig 4) receives medical data from a source in a hospital environment. In a subsequent step 51, the data is analysed to determine whether it comprises structured data. For any unstructured data, an appropriate storage policy is chosen in step 52. For any structured data, an appropriate storage policy is chosen in step 53. These steps can be carried out separately for every element of the dataset, since a dataset may comprise both structured data and unstructured data. In a subsequent step 54, the dataset is analysed to determine whether it comprises sensitive data. For any non-sensitive data, the content is indexed for search (as explained in Fig 3 above) in step 55. If sensitive data is present, this is extracted or separated in step 56 and anonymised in step 57. In a final step 58, the medical record is then stored in the cloud storage arrangement.

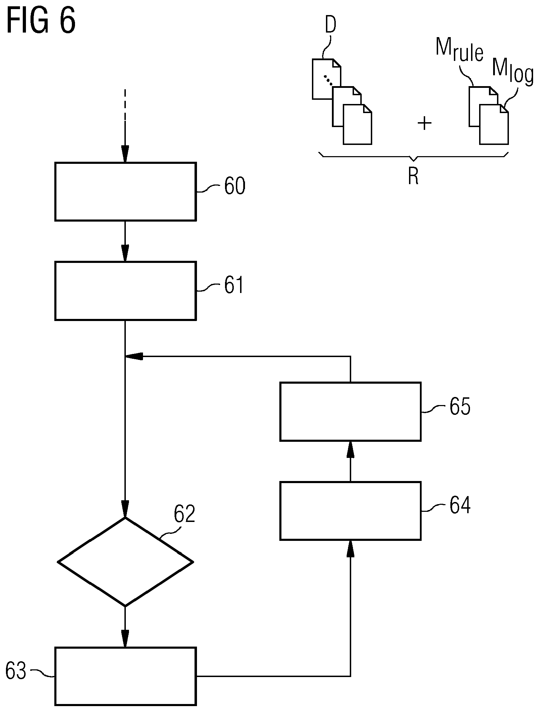

Fig 6 is a flowchart that illustrates the concept of adding a log to a medical record to track all actions performed on that medical record. The steps of this flowchart may follow any previous steps of generating metadata to be added to the medical data of a medical record. Alternatively, if further access rules are not to be added, the steps of this flowchart may follow essentially directly after data package has been uploaded to the cloud storage arrangement. Log metadata M_{log} is created for the medical data D in a first step 60 and can be embedded in the resulting medical record R, which is stored in the cloud in the subsequent step 61. At some later point in time, a request to access that medical record R may be received, as indicated by step 62. An ensuing action may be carried out in step 63, for example share the medical data, refuse access to the medical data, delete the medical record, etc.

In step 64, the nature of the action is summarized as a log entry. A log entry preferably describes the request and the action, e.g. "read request from region XYZ; access refused according to region consignment"; "delete request from owner; order to delete issued to requesting parties M, N, P; delete confirmation received from requesting parties M", etc. In step 65, the log metadata is updated with the log entry. In this way, all requests to a medical record can be tracked over time. At some point, the log metadata M_{log} of a medical record R may be used, for example in an auditing procedure, to establish whether the medical record was handled in compliance with any relevant regulation, access rule, consignment, etc.

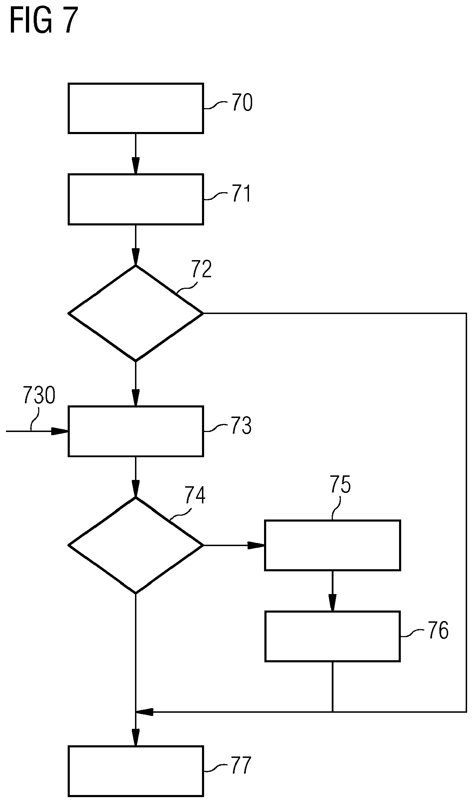

Fig 7 is a flowchart that illustrates the concept of analysing machine data. Since machine data (comprising application files and/or machine log files) can be part of the medical data uploaded to the cloud 10, the steps of this flowchart may follow essentially directly after medical data is uploaded to the cloud storage arrangement as described above. In step 70, the application files and/or machine log files are parsed using a suitable parser. The results are analysed in step 71 using a suitable analysis module. If nothing unusual or unexpected is identified in step 72, the procedure terminates at step 77. However, if an unexpected pattern is detected, a machine learning algorithm is deployed in step 73. The machine learning algorithm can be "fed" with relevant information 730, for example from a regulation module that can provide a set of any applicable regulations 730 that must be complied with (these may be laid down in a service contract, for example). In step 74 it is established whether a violation (e.g. incorrect use of a medical device, unauthorized sharing of application files, etc.) may have occurred. If not, the procedure terminates at step 77. If yes, any newly-identified "pattern" is added to a pattern database in step 75 and a report is issued in step 76, after which the process terminates in step 77.

Although the present invention has been disclosed in the form of preferred embodiments and variations thereon, it will be understood that numerous additional modifications and variations could be made thereto without departing from the scope of the invention.
For the sake of clarity, it is to be understood that the use of "a" or "an" throughout this application does not exclude a plurality, and "comprising" does not exclude other steps or elements. The mention of a "unit" or a "module" does not preclude the use of more than one unit or module.

## Claims

1. A method of managing medical records (R), which method comprises the steps of
- obtaining medical data (D) from a source (11_1, 11_2, ..., 11_n);
- generating a medical record (R) comprising the medical data (D);
- augmenting the medical record (R) with metadata (Mᵣᵤₗₑ, M_{log}), wherein the metadata (Mᵣᵤₗₑ) comprises access permissions to that medical data (D) and/or a log file for that medical record (R);
- storing the medical record (R) in a cloud storage arrangement (10), which cloud storage arrangement (10) is configured to store a plurality of medical records (R); and
- responding to a request for a medical record (R) on the basis of the metadata (Mᵣᵤₗₑ, M_{log}) of that medical record (R).

2. A method according to claim 1, wherein medical data (D) of a requested medical record (R) is released to a requesting party (12_1, 12_2, ..., 12_n) if the requesting party (12_1, 12_2, ..., 12_n) meets the requirements specified in the metadata (Mᵣᵤₗₑ) of that medical record (R).

3. A method according to claim 1 or claim 2, wherein access permissions for a medical record (R) are defined by one or more rule sets (C1, C2, C3, C4), wherein a rule set (C1, C2, C3, C4) defines a scope of access to medical data (D) of that medical record (R) at a specific access level (L1, L2, L3, L4) .

4. A method according to claim 3, wherein a rule set (C1, C2, C3, C4) specifies one or more of: an approved use of the medical data (D), the country of origin of the medical data (D), the storage duration of the medical data (D).

5. A method according to claim 3 or claim 4, wherein a rule set (C1, C2, C3, C4) comprises any of a patient consignment rule set (C1), a hospital consignment rule set (C2), a country consignment rule set (C3), a region consignment rule set (C4).

6. A method according to any of the preceding claims, comprising a step of updating the log file (M_{log}) of a medical record (R) with a log entry in response to an action relating to that medical record (R).

7. A method according to claim 6, wherein, in response to a request to delete a medical record (R), its log file (M_{log}) is examined to identify any party (12_1, 12_2, ..., 12_n) that obtained a local copy (D_{OK}) of medical data (D) of that medical record (R).

8. A method according to any of the preceding claims, further comprising a step of analysing the content of the medical data (D) and indexing the medical record (R) on the basis of its content.

9. A method according to claim 8, comprising a step of generating a master index (X1, ..., Xn) for medical records (R) stored in the cloud storage arrangement (10).

10. A method according to any of the preceding claims, further comprising a step of analysing machine data of medical apparatus (11_1, 11_2, ..., 11_n), which machine data is part of the received medical data (D).

11. A medical record management system (1) comprising
- a means of receiving medical data (D) to be stored in a medical record (R);
- a metadata augmentation module (100) realised to augment the medical data (D) with metadata (Mᵣᵤₗₑ, M_{log}) defining access permissions (Mᵣᵤₗₑ) to that medical data (D) and/or comprising a log file (M_{log}) for that medical data (D);
- a cloud storage arrangement (10) for storing the medical data (D) with its metadata (Mᵣᵤₗₑ, M_{log}) as a medical record (R); and
- a request interface (12) realised to receive a request (Q) for medical data (D) and to respond to the request (Q) on the basis of metadata (Mᵣᵤₗₑ, M_{log}) of a corresponding medical record (R).

12. A medical record management system according to claim 11, comprising an input interface (110) realised to specify an access scope (C1, C2) for that medical data (D) at an access level (L1; L2).

13. A medical record management system according to claim 11 or claim 12, wherein the metadata augmentation module (100) comprises at least one regulation module (107, 117) realised to augment a medical record (R) with regulation metadata (Mᵣᵤₗₑ).

14. A medical record management system according to any of claims 11 to 13, wherein the request manager (12) is configured to analyse the metadata (Mᵣᵤₗₑ) of stored medical records (R) in response to a request (Q) for a specific type of medical data, to identify any approvable medical records, and to release medical data (D_{OK}) of approved medical records to a requesting party (12_1, 12_2, ..., 12_n).

15. A medical record management system according to any of claims 11 to 14, comprising a data separation module realised to separate sensitive data of a medical record (R) from non-sensitive data of a medical record (R).

16. A computer program product comprising a computer program that is directly loadable into a memory of a control unit of a medical record management system (1) according to any of claims 11 to 15, and which comprises program elements for performing steps of the method according to any of claims 1 to 10 when the computer program is executed by the control unit of the medical record management system (1).
